# EUROPEAN PATENT SPECIFICATION

(11) **EP 3 125 214 B1**
(45) Date of publication and mention of the grant of the patent: **06.06.2018**
(21) Application number: 15179357.7
(22) Date of filing: 31.07.2015
(51) Int. Cl.: G09B 23/28, B25J 9/00, G06F 3/08, A61B 34/00

(54) **TOOL MANIPULATOR FOR A TRAINING AND TESTING MEDICAL DEVICE**
WERKZEUGMANIPULATOR ZUM TRAINIEREN UND TESTEN EINER MEDIZINISCHEN VORRICHTUNG
MANIPULATEUR D'OUTIL POUR DISPOSITIF MÉDICAL D'ENTRAÎNEMENT ET DE TEST

(43) Date of publication of application: 01.02.2017
(73) Proprietor: Fundacja Rozwoju Kardiochirurgii Im. Prof. Zbigniewa Religi, 41-800 Zabrze (PL)
(72) Inventor: Nawrat, Zbigniew, 41-800 Zabrze (PL); Mucha, Lukasz, 37-700 Przemysl (PL); Lehrich, Krzysztof, 43-450 Ustron (PL); Lis, Krzysztof, 42-674 Wilkowice (PL); Rohr, Kamil, 41-933 Bytom (PL)
(74) Representative: Malcherek, Piotr

(56) References cited:
- WO-A1-01/87550
- WO-A1-86/00039
- WO-A1-2006/066401
- US-A1- 2004 024 387

## Description

### THE INVENTION

The invention relates to a training and testing medical device which enables an operator to develop manual skills in relation to a specific medical instrument. By applying said training and testing medical device it is also possible to test remote-controlled surgical robots. In both of the aforesaid applications the device according to the invention enables to test and monitor motion parameters of a human-controlled surgical instrument or a tool controlled by a manipulator, wherein it is also possible to generate force interaction on the controlled surgical instruments which allows to create force feedback thus providing the operator with a subjective sense of contact between the controlled object and a preset obstacle located in the working area of the medical instrument.

### STATE OF THE ART

There are known solutions relating to manipulators - motion controllers which are used for controlling medical devices while also providing feedback between the controlled object and an obstacle located within the working area of said controlled object. The constructions known to date do not enable operators to develop their manual skills or to test tool manipulators in relation to a specific surgical instrument.

CN 103111998 discloses a manipulator which is a motion controller comprising two platforms - a fixed platform and a mobile platform. The fixed platform constitutes a part of the motion controller which is devoid of all degrees of freedom and which remains immobile in relation to other subassemblies of the manipulator in operation, that is while the mobile platform is moving. The mobile platform is a mobile element which can change its position in relation to the fixed platform by following preset trajectories in its working area. On the fixed platform there are three motors and encoders, that is measuring devices - rotational pulse transducers which enable the measurement of rotation angles. Lines going through axes of rotation of the motor shafts intersect at an angle of 60 ° and form an equilateral triangle. Both platforms are connected by means of three connecting assemblies in the form of rods. Said connecting rods are mounted on both sides in the Cardan joint, which results in the possibility to perform two rotations on each side of the rod. Torque from the connecting rods is transferred onto the motor shaft by an intermediary element in the form of a semicircle pressed down by means of a strand. This requires sufficient tension of the strand, which results in elaboration of the entire mechanism. Kinematics based solely on Cardan joints leads to limited mobility. Said motion controller can work only in one position due to gravitational compensation in the gripping part. Said manipulator provides the user with force feedback, that is a subjective sense of contact and force exerted on an element e.g. tool by generating appropriate force in a certain direction affecting the operator.

Moreover, in the prior art there are commonly known solutions based on parallel structures and so-called Delta structures. For example, such solution is disclosed in EP 1876505 B1, in which the base of the manipulator is connected to the gripping part by means of a kinematic mobile system. The connection between the base of the manipulator and the gripping part ensures three degrees of freedom of motion of said gripping part while maintaining a constant, preset distance between the gripping part and the base of the manipulator. The construction also has passive gravity compensation means of the gripping part which also enable to exert force or torque on the gripping part. In the construction connecting the base with the gripping part there is also a built-in actuator adapted to move with the structure within three degrees of freedom and for actuating the gripping part along at least one degree of freedom.

### THE AIM OF THE INVENTION

The aim of the present invention is to provide a training and testing medical device enabling an operator of a medical device to develop manual skills as well as to test medical robots by monitoring motion parameters of a medical instrument including testing motion trajectories of a medical instrument within the space of the device, setting and monitoring the location of the fixed point of a medical instrument while ensuring a subjective sense of contact between the controlled medical instrument and a preset obstacle within the working area of the training and testing device and measuring the force exerted on the medical instrument as well as the force exerted by the controlled tool on the preset obstacles. Another aim of the invention is to provide such a construction of the training and testing device that it can be mounted in any position enabling unconstrained motion of the medical instrument while ensuring as large a working area as possible within which the medical instrument can be moved freely.

### THE ESSENCE OF THE INVENTION

The present invention relates to a training and testing medical device comprising a fixed platform mounted on an extension arm, wherein on said fixed platform there are motors with encoders and a mobile platform connected thereto. The invention is characterised by the fact that on said fixed platform there are six motors with encoders, wherein shafts of said three motors with encoders are connected to said first mobile platform by means of connecting assemblies, whereas shafts of the remaining three motors with encoders are connected to a second mobile platform by means of connecting assemblies. Each connecting assembly comprises a first arch connector with an angle range of 90 degrees whose first end is directly rigidly connected to the shaft of the respective motor with an encoder and the second end is connected by means of a joint with three degrees of freedom to a first end of a second arch connector, whereas the second end of the second arch connector has at its end a transverse arch arm and is rotatably connected to the respective mobile platform. Each mobile platform has a form of three rings for mounting therein a holder of a medical instrument. Said holder of a medical instrument is rotatably and non-slidably mounted in the rings of each mobile platform. The holder of a medical instrument mounted in the two mobile platforms has a form of a telescopic pipe element. At the same time the lines comprising axes of rotation of the shafts of each group of the three motors with encoders intersect at one point at an angle of 120 degrees.

Preferably the shafts of the three motors with encoders are directed outward of the fixed platform and connected to the first mobile platform by means of connecting assemblies, whereas the shafts of the remaining three motors with encoders are directed inward the fixed platform and are connected to the second mobile platform by means of connecting assemblies.

It is preferable that the axis of each rotational connection between the arch arm and the mobile platform be perpendicular to the axis of the holder of a medical instrument. The axis of the first rotational connection and the axes of the other two rotational connections in the mobile platform intersect at one point on the axis of rotation of the medical instrument holder.

It is preferable that at least one mobile platform be equipped with a drive with an encoder for measuring the rotation of the holder of a medical instrument around its own axis.

It is also advisable that the fixed platform have two levels, wherein on the first level there should be three motors with encoders with shafts directed outward the fixed platform and the remaining three motors with encoders with shafts directed inward the fixed platform be located on the second level of said fixed platform.

### ADVANTAGES OF THE INVENTION

The main advantage of the training and testing medical device is the fact that with the use of one medical device it is possible to develop the operator's manual skills as well as test tool manipulators, including medical robots. Another advantage of the invention relates to providing an operator with a subjective sense of contact with an obstacle, therefore providing gravitational compensation with feedback while maintaining the device relatively simple in terms of construction. Still another advantage of the invention is that the device and the medical instrument mounted thereon have extensive motion capabilities, which results in a large working area of the medical instrument, and the movements of the operator or of the tested medical manipulator are not constrained by parts of the device.

### DESCRIPTION OF THE DRAWINGS

The invention is presented in more detail in the following embodiments shown in the drawing, where:
- Fig. 1: is a top view of the medical device with marked lines comprising shafts of the motors with encoders,
- Fig. 2: is a top view of the medical device with a marked angular profile of the arch connector,

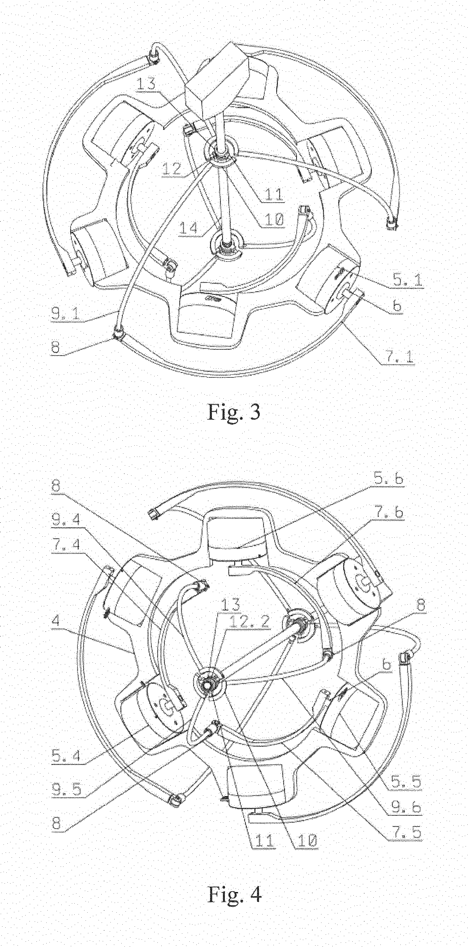

- Fig. 3: is a perspective view of the medical device,
- Fig. 4: is another perspective view of the medical device,

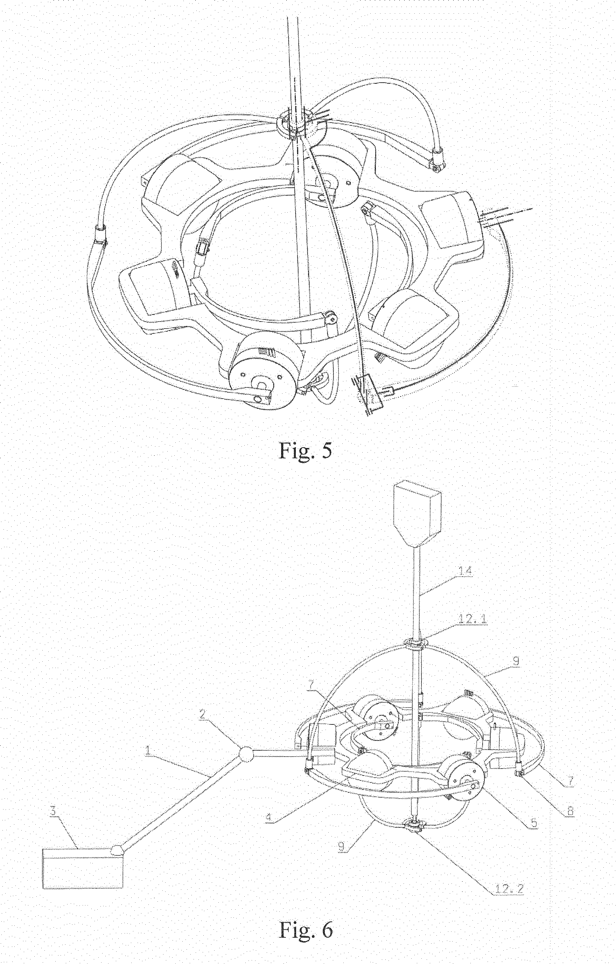

- Fig. 5: is a perspective view of the medical device with a marked motion path of the connecting assembly,
- Fig. 6: is a perspective view of the medical device with the extension arm and base shown,

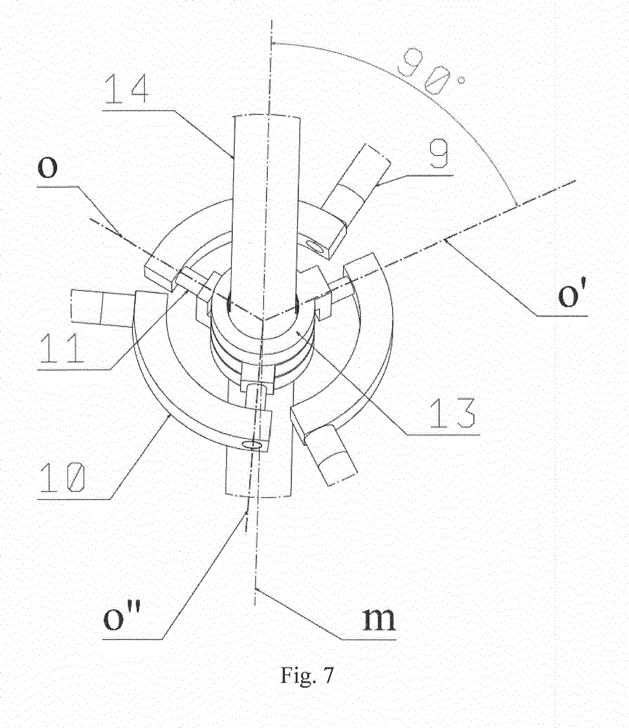

- Fig. 7: is a detailed perspective view of the mobile platform,
- Fig. 8: is a schematic perspective view of the medical device with the mobile platform equipped with a drive,
- Fig. 9: is a detailed perspective view of the mobile platform equipped with a drive,
- Fig. 10: is a detailed view of the mobile platform with a drive with a partial sectional view of the rings,
- Fig. 11: is a perspective view of the medical device with a second mobile platform equipped with a drive,

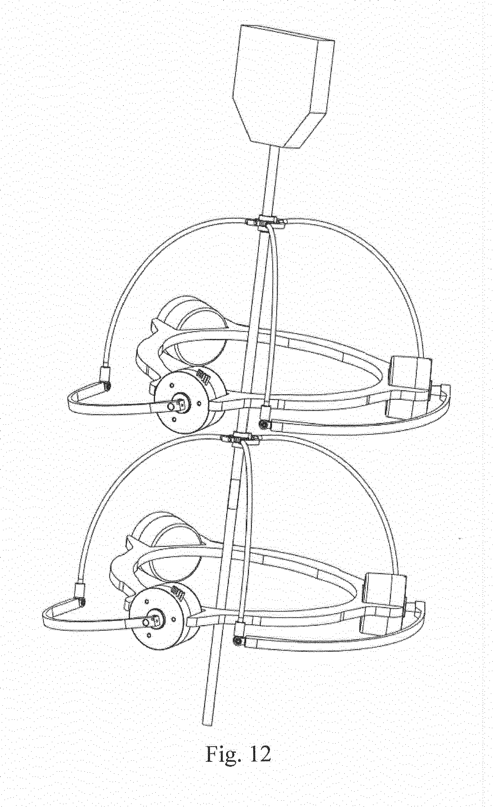

- Fig. 12: is a schematic perspective view of the medical device with a two-level fixed platform,
- Fig. 13: is a kinematic chart of the device according to the invention.

### EMBODIMENTS

The training and testing medical device comprises an extension arm 1 on one side mounted on a base 3 in any manner, and connected to a fixed platform 4 on the other side. The extension arm 1 has any number of joints 2 enabling an angular change in the position of the extension arm 1, which results in the possibility of arranging the medical device in any position depending on the user's preference.

On the fixed platform 4, on one level, there are six motors with encoders 5, wherein shafts 6 of the three motors with encoders 5.1, 5.2, 5.3 are directed outward of the fixed platform 4, whereas shafts 6' of the remaining three motors with encoders 5.4, 5.5, 5.6 are directed inward the fixed platform 4. The three motors with encoders 5.1, 5.2, 5.3 are so arranged on the fixed platform 4 that lines comprising axes of rotation of the shafts 6 of said motors intersect at one point at an angle of 120 degrees. The remaining three motors with encoders 5.4, 5.5, 5.6 are so arranged that the lines comprising the axes of rotation of the shafts 6' of said motors intersect at one point at an angle of 120 degrees. The shafts 6 of the three motors with encoders 5.1, 5.2, 5.3 are connected to a first mobile platform 12.1 by means of connecting assemblies. Each connecting assembly comprises a first arch connector 7 with an angle range of 90 degrees. The first end of said connector 7 is directly and rigidly connected to the shaft 6 of the respective motor with an encoder 5.1, 5.2, 5.3, and the second end of said connector 7 is connected to a first end of a second arch connector 9 by means of a joint 8. The joint 8 has three degrees of freedom. The second end of the second arch connector 9 has at its end a transverse arch arm 10 and is rotatably 11 connected to the first mobile platform 12.1. The mobile platform 12.1 has a form of three rings 13 arranged one on top of the other and is provided for mounting thereon a holder 14 of a medical instrument. The shafts 6' of the remaining three motors with encoders 5.4, 5.5, 5.6 are directed inward the fixed platform 4 and connected to a second mobile platform 12.2 by means of connecting assemblies. Each connecting assembly comprises a first arch connector 7 with an angle range of 90 degrees. The first end of said connector 7 is directly and rigidly connected to the shaft 6' of the respective motor with an encoder 5.4, 5.5, 5.6, and the second end of said connector 7 is connected to a first end of a second arch connector 9 by means of a joint 8. The joint 8 has three degrees of freedom. The second end of the second arch connector 9 has at its end the transverse arch arm 10 and is rotatably 11 connected to the second mobile platform 12.2. The mobile platform 12.2 has a form of three rings 13 arranged one on top of the other and is provided for mounting thereon the holder 14 of a medical instrument. The rings may rotate in relation to each other to a limited extent.

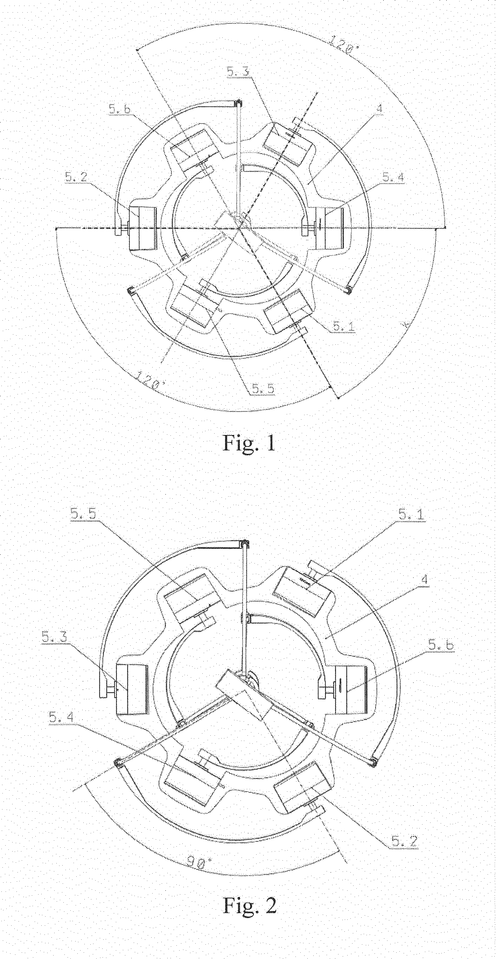

As it has been disclosed above, in each connecting assembly the first arch connector 7 has an angle range of 90 degrees, by which it is understood that the connector 7 is so shaped that the axis of rotation of the joint 8 located on the second end of said connector 7 is arranged at an angle of 90 degrees in relation to the axis of rotation of the shaft 6, 6' of the motor with an encoder 5 to which the first end of said connector 7 is connected (fig.2).

The holder 14 of a medical instrument has a form of a telescopic pipe element, which allows to mount thereon medical instruments of different lengths as well as to arrange the medical instrument at any height and uncomplicated adjustments of the height reflecting normal mode of work with the use of a medical instrument.

Each mobile platform 12.1, 12.2 is mounted on the holder 14 of a medical instrument in a fixed point. Each ring 13 forming the mobile platforms 12.1, 12.2 is rotatably 11 connected with the arch arm 10 of the connecting assembly. The connection is achieved in such a way that the axes o, o', o" of each rotatable connection 11 are perpendicular to the axis m of the holder 14 of a medical instrument, and said axes o, o', o" intersect at one point on the axis m of rotation of the holder 14 of a medical instrument. It has been achieved owing to the fact that the points of rotatable connection 11 in the three rings 13 are located on one level of the middle ring 13. In the case of the top ring 13 the point of rotatable connection 11 was lowered, whereas in the case of the bottom ring 13 the point of rotatable connection 11 was raised, and in order to achieve the above suitably shaped protrusions on the circumferences of said rings 13 were used. This ensures stable mounting of the mobile platforms 12.1, 12.2 on the holder 14 of a medical instrument while maintaining considerable freedom of motion. The medical instrument itself is rotatably mounted on the holder 14, which ensures rotation of a medical instrument around its axis.

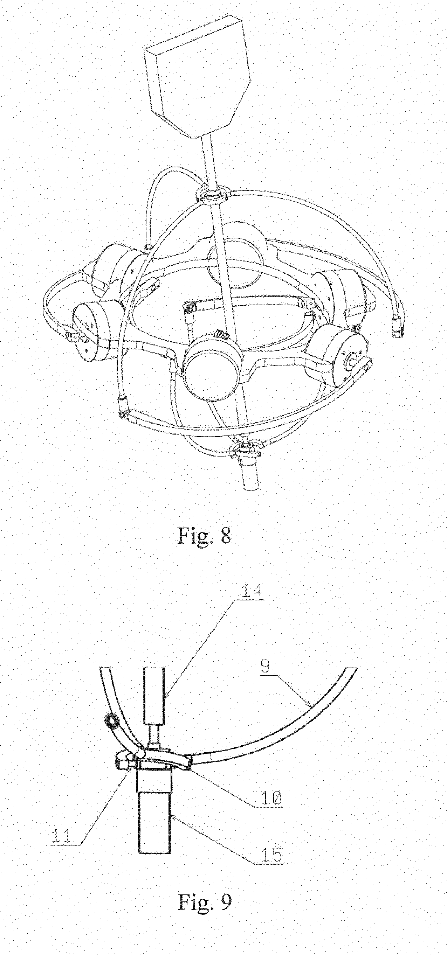

Another possible embodiment of the training and testing medical device according to the invention consists in that one of the mobile platforms 12.1, 12.2 is equipped with a drive 15 (fig. 8, 9). The drive 15 is equipped with an encoder allowing to measure rotation and generating forces, for example when the medical instrument rotates around its own axis. The drive 15 is immovably mounted on the mobile platform 12.1, 12.2 in the point of the rotatable connection 11 with the arch arm 10.

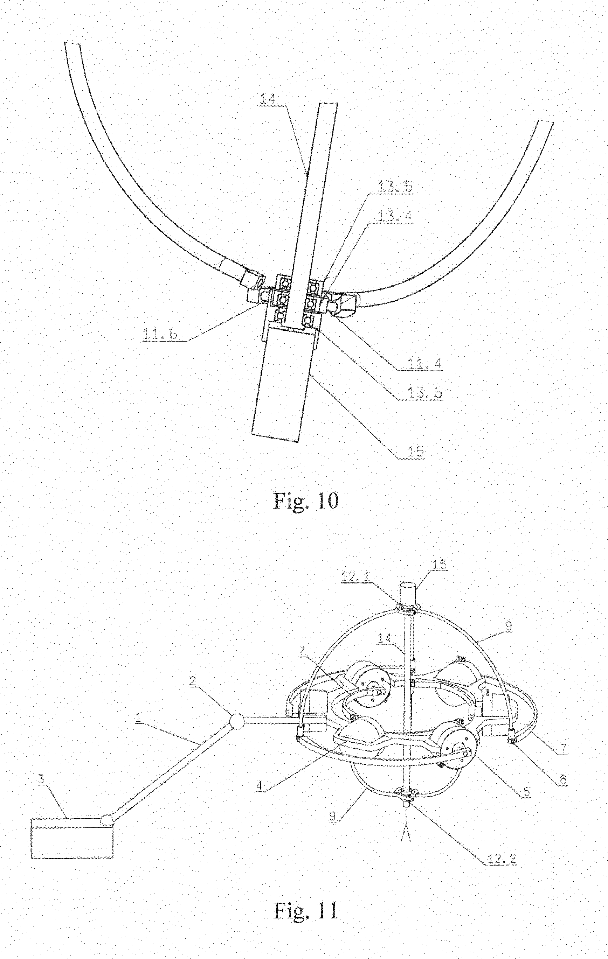

In another embodiment (fig. 10) the fixed platform 4 of the medical training and testing device can comprise two levels. On each level there are three motors with encoders 5, wherein said motors arranged on one level of the fixed platform 4 are assigned to a respective mobile platform.

In each of the embodiments the medical instrument fastened in the holder may be arranged in any position in relation to the mobile platforms 12.1, 12.2. They may be arranged below the fixed point of the medical instrument or above this point. The mobile platforms 12.1, 12.2 may also be so arranged that one of said platforms is located below and the other above the fixed point of the medical instrument. One of the mobile platforms 12.1, 12.2 may also be arranged within the fixed point of the medical instrument, therefore it may maintain the fixed point for the medical instrument in a fixed position, while the other mobile platform 12.1, 12.2 will record movement of the instrument in space.

It must be explained that appropriate software is used for recording changes in the position of encoders, thus for monitoring various motion parameters as well as for achieving gravitational compensation and feedback. The software allows to record the position of the instrument in space by transforming signal provided from detectors measuring angular arm deflection. Owing to this an operator may control the virtual and actual robot in the operating theatre. The software also enables to exert force on the tool-operator with the use of motors, by securing the instrument in a certain position, by generating a resistance of a preset force in any selected direction, which provides the operator with a sense of contact with an obstacle.

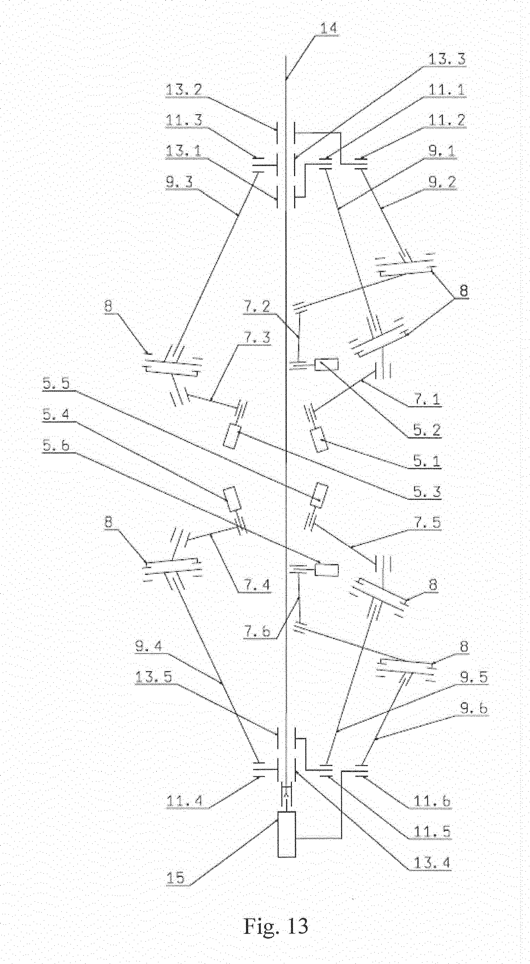

Fig. 13 is a kinematic chart of the device according to the invention.

## Claims

1. A training and testing medical device comprising a fixed platform (4) mounted on an extension arm (1) and a mobile platform (12.1) connected to the fixed platform (4) by means of connecting assemblies, the fixed platform (4) comprising motors with encoders (5), **characterised in that** there are six motors with encoders (5) on the fixed platform (4) wherein shafts (6) of three of said six motors (5.1, 5.2, 5.3) are connected to said first mobile platform (12.1) by means of connecting assemblies, and shafts of the remaining three motors with encoders (5.4, 5.5, 5.6) are connected to a second mobile platform (12.2) by means of connecting assemblies, and each connecting assembly comprises a first arch connector (7) with an angle range of 90 degrees, whose first end is directly and rigidly connected to the shaft (6, 6') of the respective motor with an encoder (5.1, 5.2, 5.3, 5.4, 5.5, 5.6), and whose second end is connected by means of a joint (8) with three degrees of freedom to a first end of a second arch connector (9), whereas the second end of the second arch connector (9) has at the end thereof a transverse arch arm (10) and is rotatably connected (11) to the respective mobile platform (12.1, 12.2), wherein each mobile platform (12.1, 12.2) has a form of three rings (13.1, 13.2, 13.3, 13.4, 13.5, 13.6) for mounting therein a holder (14) of a medical instrument, wherein the holder (14) of a medical instrument is rotatably and unslidably mounted in the rings (13) of the mobile platforms (12.1, 12.2), wherein the holder (14) of a medical instrument has a form of a telescopic pipe element, and lines comprising axes of rotation of the shafts (6, 6') of each group of the three motors with encoders (5) intersect at one point at an angle of 120 degrees.

2. The device according to claim 1, **characterised in that** the shafts (6) of the three motors with encoders (5.1, 5.2, 5.3) are directed outward of the fixed platform (4) and are connected to said first mobile platform (12.1) by means of connecting assemblies, and the shafts (6') of the remaining three motors with encoders (5.4, 5.5, 5.6) are directed inward the fixed platform (4) and are connected to the second mobile platform (12.2) by means of connecting assemblies.

3. The medical device according to claim 1 or 2, **characterised in that** in the mobile platform (12.1, 12.2) the axis (o, o', o") of each rotational connection (11) is perpendicular to the axis (m) of rotation of the holder (14) of a medical instrument, and the axis (o) of the first rotational connection (11) and the axes (o', o") of the remaining two rotational connections (11) in the mobile platform (12.1, 12.2) intersect at one point on the axis (m) of rotation of the holder (14) of a medical instrument.

4. The medical device according to one of the claims 1-3, **characterised in that** at least one mobile platform (12.1, 12.2) is equipped with a drive (15) with an encoder immovably connected in the point of the rotational connection (11) with the arch arm (10).

5. The medical device according to one of the claims 1-4, **characterised in that** the fixed platform (4) has two levels, wherein on the first level there are three motors with encoders (5) with the shafts (6) directed outward of the fixed platform (4), and the remaining three motors with encoders (5) with the shafts (6') directed inward the fixed platform (4) are arranged on the second level of said fixed platform (4).

## Patentansprüche

1. Medizinische Training-Test-Vorrichtung mit einer an einem Verlängerungsarm (1) montierten festen Plattform (4), die mit Motoren mit Encodern (5) versehen ist, sowie mit dieser mittels Verbindungsanordnungen verbundenen mobilen Plattform (12.1), **dadurch gekennzeichnet, dass** auf der festen Plattform (4) sechs Motoren mit Encodern (5) vorgesehen sind, wobei die Wellen (6) von drei von sechs Motoren mit Encodern (5.1, 5.2, 5.3) jeweils mittels Verbindungsanordnungen mit der gegenständlichen ersten mobilen Plattform (12.1) verbunden sind, und die Wellen der anderen drei Motoren mit Encodern (5.4, 5.5, 5.6) mittels Verbindungsanordnungen mit der zweiten mobilen Plattform (12.2) verbunden sind, und jede Verbindungsanordnung einen ersten Bogenverbinder (7) mit einem Winkelbereich von 90° umfasst, dessen erstes Ende direkt starr mit der Welle (6, 6') des zugehörigen Motors mit Encoder (5.1, 5.2, 5.3, 5.4, 5.5, 5.6) verbunden ist, und dessen zweites Ende mittels eines Gelenks (8) mit drei Freiheitsgraden an das erste Ende eines zweiten Bogenverbinders (9) angeschlossen ist, hingegen ein zweites Ende des zweiten Bogenverbinders (9) einen Querbogenarm (10) aufweist und über eine Drehverbindung (11) mit der zugehörigen mobilen Plattform (12.1, 12.2) drehbar verbunden ist, wobei jede mobile Plattform (12.1, 12.2) eine Form von drei Ringen (13.1, 13.2, 13.3, 13.4, 13.5, 13.6) aufweist, welche zum Einbetten eines Greifteils (14) eines medizinischen Instrumentes vorgesehen sind, wobei das Greifteil (14) des medizinischen Instruments in Ringen (13) der mobilen Plattformen (12.1, 12.2) drehbar und nicht verschiebbar eigebettet ist und eine Form von einem teleskopisch ausziehbaren Rohrelements aufweist, und die Geradlinien, die die Drehachsen der Wellen (6, 6) jeder Drei-Motoren-Gruppe mit Encodern (5) umfassen, sich an einem Punkt in einem Winkel von 120° durchschneiden.

2. Medizinische Vorrichtung nach Anspruch 1 **dadurch gekennzeichnet, dass** die Wellen (6) von drei Motoren mit Encodern (5.1, 5.2, 5.3) außerhalb der festen Plattform (4) gerichtet und durch Verbindungsanordnungen mit der ersten gegenständlichen mobilen Plattform (12.1) verbunden sind, hingegen die Wellen (6') der übrigen drei Motoren mit Encodern (5.4, 5.5, 5.6) innenhalb der festen Plattform (4) gerichtet und durch Verbindungsanordnungen mit der zweiten mobilen Plattform (12.2) verbunden sind.

3. Medizinische Vorrichtung nach Anspruch 1 oder 2, **dadurch gekennzeichnet, dass** in der mobilen Plattform (12.1, 12.2) eine Achse (o, o', o") jeder Drehverbindung (11) senkrecht zur Drehachse (m) des Greifteils (14) des medizinischen Instruments verläuft, während die Achse (o) der ersten Drehverbindung (11) und die Achsen (o', o") der beiden anderen Drehverbindungen (11) sich in der mobilen Plattform (12.1, 12.2) an einem auf der Drehachse (m) des Greifteils (14) des medizinisches Instruments liegenden Punkt durchschneiden.

4. Medizinische Vorrichtung nach einem der Ansprüche 1 - 3, **dadurch gekennzeichnet, dass** mindestens eine mobile Plattform (12.1, 12.2) mit einem Antrieb (15) mit Encoder ausgestattet ist, welcher mit dem Querbogenarm (10) im Punkt der Drehverbindung (11) starr verbundenen ist.

5. Medizinische Vorrichtung nach einem der Ansprüche 1 - 4, **dadurch gekennzeichnet, dass** die feste Plattform (4) zwei Ebenen aufweist, wobei auf einer ersten Ebene drei Motoren mit Encodern (5) mit zur Außenseite der festen Plattform (4) ausgerichteten Wellen (6) angeordnet sind, und die übrigen drei Motoren mit Encodern (5) mit zur Innenseite der festen Plattform (4) gerichteten Wellen (6') auf einer zweiten Ebene dieser festen Plattform (4) angeordnet sind.

## Revendications

1. Dispositif médical de formation et d'essai contenant une plate-forme fixe (4) montée sur une flèche (1) sur laquelle sont placés les trois moteurs avec les encodeurs (5) et une plate-forme mobile (12.1) connectée à la plate-forme fixe (4) à l'aide d'assemblages reliant **caractérisé en ce que** sur cette plate-forme fixe (4) il y a six moteurs avec les encodeurs (5), où les arbres (6) de trois des six moteurs avec les encodeurs (5.1, 5.2, 5.3) sont connectés à l'aide d'assemblages reliant à ladite première plate-forme mobile (12.1) et les arbres de trois autres moteurs avec les encodeurs (5.4, 5.5, 5.5) sont connectés à la deuxième plate-forme mobile (12.2) à l'aide d'assemblages reliant, et chaque assemblage reliant comprend un premier connecteur en forme d'arc (7) de la plage angulaire de 90 degrés dont la première extrémité est directement connectée rigidement à l'arbre (6, 6') du moteur avec l'encodeur lui affecté (5.1, 5.2, 5.3, 5.4, 5.5, 5.6) et l'autre extrémité est connectée à l'aide du joint (8) à trois degrés de liberté à la première extrémité du deuxième connecteur en forme d'arc (9) et l'autre extrémité du deuxième connecteur en forme d'arc (9) est terminée par le bras transversal en forme d'arc (10) et est reliée de façon rotative (11) à la plate-forme mobile lui associée (12.1,12,2) pendant que chaque plate-forme mobile (12.1, 12.2) a la forme de trois anneaux (13.1, 13.2, 13.3. 13.4, 13.5, 13.6) destinés à y encastrer une poignée (14) d'un outil médical pendant que la poignée d'un outil médical est encastrée dans les anneaux (13) de plates-formes mobiles (12.1, 12.2) de façon rotative et non coulissante pendant que la poignée (14) d'un outil médical possède une forme d'élément tubulaire télescopique et les droites contenant les axes de rotation d'arbres (6, 6') de chaque groupe de trois moteurs avec les encodeurs (5) se coupent en un point à un angle de 120 degrés.

2. Dispositif médical selon la revendication 1 **caractérisé en ce que** les arbres (6) de trois moteurs avec les encodeurs (5.1, 5.2, 5.3) sont dirigés vers l'extérieur de la plate-forme fixe (4) et sont connectés à la première plate-forme mobile (12.1) à l'aide d'assemblages reliant et les arbres (6') de trois autres moteurs avec les encodeurs (5.4, 5.5, 5.6) sont dirigés vers l'intérieur de la plate-forme fixe (4) et sont connectés à la deuxième plate-forme mobile (12.2) à l'aide d'assemblages reliant.

3. Dispositif médical selon la revendication 1 ou 2 **caractérisé en ce que** l'axe (o, o', o") de la plate-forme mobile (12.1, 12.2) pour chaque connexion rotative (11) est perpendiculaire à l'axe (m) de rotation de la poignée (14) de l'outil médical et simultanément l'axe (o) de la première connexion rotative (11) et les axes (o', o") de deux autres connexions de la plate-forme mobile (12.1, 12.2) se coupent en un point situé sur l'axe (m) de rotation de la poignée (14) de l'outil médical.

4. Dispositif médical selon l'une des revendications 1 - 3 **caractérisé en ce qu'**au moins une plate-forme mobile (12.1, 12.2) est équipée d'une source d'entraînement (15) avec l'encodeur relié de façon immobile au point de la connexion rotative (11) au bras en forme d'arc (10).

5. Dispositif médical selon l'une des revendications 1-4 **caractérisée en ce que** la plate-forme fixe (4) comporte deux niveaux, sur le premier niveau se situent les trois moteurs avec les encodeurs (5) dont les arbres (6) sont dirigés vers l'extérieur de la plate-forme fixe (4) et les trois autres moteurs avec les encodeurs (5) dont les arbres (6') sont dirigés vers l'intérieur de la plate-forme fixe (4) et ils se situent sur le deuxième niveau de la plate-forme fixe (4).
